# EUROPEAN PATENT SPECIFICATION

(11) **EP 2 281 529 B1**
(45) Date of publication and mention of the grant of the patent: **08.02.2012**
(21) Application number: 09382130.4
(22) Date of filing: 30.07.2009
(51) Int. Cl.: A61F 2/00

(54) **Surgical mesh**
Chirurgisches Netz
Maille chirurgicale

(43) Date of publication of application: 09.02.2011
(73) Proprietor: Zaragoza Fernández, Cristóbal, 46183 L'Eliana - Valencia (ES); Marin Morales, Juan, 41011 Sevilla (ES); Navarro, Francisco, 34080 Montpellier (FR); Fatás Cabeza, José Antonio, 50006 Zaragoza (ES)
(72) Inventor: Zaragoza Fernández, Cristóbal, 46183 L'Eliana - Valencia (ES); Marin Morales, Juan, 41011 Sevilla (ES); Navarro, Francisco, 34080 Montpellier (FR); Fatás Cabeza, José Antonio, 50006 Zaragoza (ES)
(74) Representative: Dupuis, François

(56) References cited:
- EP-A1- 1 595 512
- US-A1- 2001 049 538
- US-A1- 2006 253 203
- US-B1- 6 241 768

## Description

### Technical field of the invention

The present invention refers to a surgical mesh for the treatment of hernia, particularly inguinal region hernias.

### Background and state of the art

An inguinal hernia is a condition in which a portion of intraabdominal viscera (generally epiploon, small intestine or large intestine) protrudes through a weak place or defect inside the lower abdominal muscular wall or groin of a patient. This condition occurs commonly in human beings (both men and women). This kind of hernia can be a congenital defect in which the patient is born with this problem or can be caused by the permanent force of the intraabdominal pressure, because of an instantaneous pressure of great force (efforts, lifting heavy objects or due to an abdominal closed trauma) or muscular tissue weakness (aging, degenerative processes, medications...). If the hernia is not serious, the patient could be left with an unsightly intraabdominal tissue lump that protrudes through the defect, pain and reduced lifting abilities. However, operating is usually preferred because the natural evolution of the process (due to the constant intraabdominal pressure) leads to hernial orifice enlargement and therefore, to the hernia enlargement; the patient must be operated and urgently if the hernial orifice diameter comes to conflict with the hernia vascularization (strangulated hernia) because blood flow stops and viscera necrosis is originated.

Inguinal hernia repair is one of the most common operations in general surgery. This surgical procedure generally involves the access and examination of the defect, the careful pushing of the abdominal content through the defect back to the patient's abdomen and the defect's closing. Nevertheless, nowadays we have many surgical techniques, and there is not an unanimous agreement amongst surgeons on which one is better and fundamentally on which one has the lowest recidive rate, one of this operation's big problems. Since Bassini, at the end of XIX century, introduced his hernia repair method by direct suture, both the variants of the original technique (Shouldice) and the new techniques that require placing a mesh (Lichtenstein, Gilbert, etc.) have been numerous. In addition, besides the conventional open surgery, the number of surgical options to treat this condition has increased thanks to the laparoscopic surgery, in both its intraperitoneal and preperitoneal variants.

Results have been improved by patch implantation made to reinforce classical repairs, reducing hernia reappearance to a large extent. The recidive can be ascribed to several factors such as a wrong mesh placing, mesh migration, distortion or fold; its suture and fastening to the wall can cause a wrong tension distribution; dead space creation and fluids accumulation; hematoma that can displace the prosthesis; spermatic cord preparation and placing; inadequate mesh' shape and size; low or moderate fibroplastic infiltration; etc.

The most effective implantation in mesh or prosthesis placing is obtained following the principles of the sutureless tension-free repair technique. It usually requires the use of mesh prosthesis with a communicating orifice and a groove for the spermatic cord (man) or the round ligament (woman) insertion. This kind of prosthesis placing does not cause tissue tension, but it still has some drawbacks such as the possible migration of the mesh, postoperative pain (immediate due to the big quantity of muscular suture or chronic because of nervous entrapment), it can damage the spermatic duct and it does not solve the recidive problem completely.

Among the materials that are used for the mesh, the monofilament polypropylene stands out these days due to its good tolerance, its distortion resistance and its stimulation of colonization by fibroblasts. There are some well-known monofilament polypropylene products in the market, such as Marlex^{®} (Bard), Prolene^{®} (Ethicon), Trelex^{®} (Meadox), etc. There are also some fibers with similar properties, like the Gore Tex^{®}.

The document EP 0 827 724 A2 describes a hernioplasty prosthesis made of a mesh of monofilament polypropylene preformed in a finger-nail shape with central hole and groove for the spermatic cord.

The document US-A-20030171823 describes a bidimensional mesh implant from two mesh layers that surround a central orifice, and that present respectively each layer an access groove to its central orifice that interrupts the annular shape. Both mesh layers are superimposed on the central orifices essentially aligned and with the access grooves shifted, and in relation to the peripheral direction, just attached firmly to each other in a common access side of the grooves.

The document EP 1 673 030 A1 describes a bidimensional mesh implant for the hernia treatment which has two layers of mesh with their central orifices aligned and their access grooves in shifted position in an angle of 180°. Both layers are identically manufactured and have congruent shapes.

However, most of the meshes for the inguinal hernioplasty used until now in the state of the art only repair the preperitoneal space or the inguinal canal floor; the few meshes which repair both spaces must be modified by the surgeon, or they have mechanisms that are complex to manipulate, or they implant too much foreign tissue that causes an inflammatory response and possible painful sequelae. Many of them need to be fixed with wide sutures that aggravate the problems of pain, nerve injury and foreign body.

The documents US 6,241,768 and US 6,425,924 B1 describe prosthesis or meshes that are inserted in the preperitoneal space and the inguinal floor and occlude the internal inguinal orifice, but they have several disadvantages.

The mesh described in US 6,241,768 B1 is made up of several independent stuck tissue sheets (it is not "monoblock"), it has a rigid central cylinder (even palpable in the postoperative in slim patients) and it is closed on one side, so it does not allow the spermatic cord or the round ligament to pass, and they are displaced. In addition, it does not have a lateral opening, therefore we must make a cut on the mesh with the consequent risk of later tearing. This drawbacks are described in medical literature.

The mesh described in US 6,425,924 B1 is also made up of several independent parts, neither allows the spermatic cord or ligament to pass, it needs a manipulation (tighten a thread which joins the different parts) once partially placed and also needs to be cut in its upper surface.

Although it is accepted that most of the previous meshes could be implanted without suturing them to the patient's tissues, there is a high risk of migration in all of them if they are not sutured.

Consequently, it would be desirable to find a mesh prosthesis which would solve all or some of the problems of the state of the art, especially, that could correct the hernial defect in the preperitoneal space as well as in the inguinal canal floor simultaneously and that also would occlude the inguinal orifice, implanting as little tissue as possible, allowing the cord or ligament to pass without cutting or breaking the prosthesis and needing the less possible number of sutures to the patient's tissue.

### SUMMARY OF THE INVENTION

The inventors have found that the use of a catenoid-shaped mesh makes it possible to simultaneously repair the hernial defect in the preperitoneal space and the inguinal canal floor. Moreover, the catenoid shape enables a correct mesh fastening without suture or with a minimum suture, and adds the simplicity of plug techniques.

Likewise, the mesh has a lateral opening that enables its implantation by means of a very simple hernioplasty tension-free surgical technique. Besides, the mesh presents a light structure made, on one side, of non-reabsorbing fibers that guarantee the shape maintenance and the colonization by fibroblasts in order to get the greatest adhesion, and on the other side, of absorbing fibers that form a low-weight tissue which does not cause response or foreign body sensation.

Furthermore, its lateral opening makes it possible not to cut the prosthesis to place the spermatic cord (4) or the round ligament and allows them to pass through their anatomical way preventing their displacement or compression.

Thus, in a first aspect, the invention refers to a catenoid-shape mesh that comprises:
a) an elliptical-shaped upper side (1)
b) a circular-shaped lower side (2)
leaving a non-connected lateral space between both sides (3) and a central gap (3').

The size of the body surface, and hence the size of the inguinal region, varies depending on the sex, age and ethnic group of the patient. Consequently, most of the meshes in the state of the art need to be manipulated by the surgeon, for example, being cut in order to reduce their surface, with the consequent risk of later tearing. However, the special catenoid shape of the mesh of the invention enables its placing and adaptation in patients of diverse morphology.

According to a particular embodiment, the size of the upper surface ellipse (1) is characterized by having a longitudinal axis from 13 cm to 17 cm long and a transverse axis from 8 cm to 12 cm long. In a more particular embodiment, the ellipse longitudinal axis size is 15 cm. According to another more particular embodiment, the ellipse transverse axis size is 10 cm. In an even more particular embodiment, the ellipse longitudinal axis is 15 cm long and the transverse axis is 10 cm long.

According to another particular embodiment, the axis (diameter) of the lower surface (2) is from 4 cm to 6 cm long, more particularly is 5 cm long.

In another particular embodiment, the communicating orifice (3') diameter length is from 1cm to 2 cm, and more particularly is 1.5 cm long.

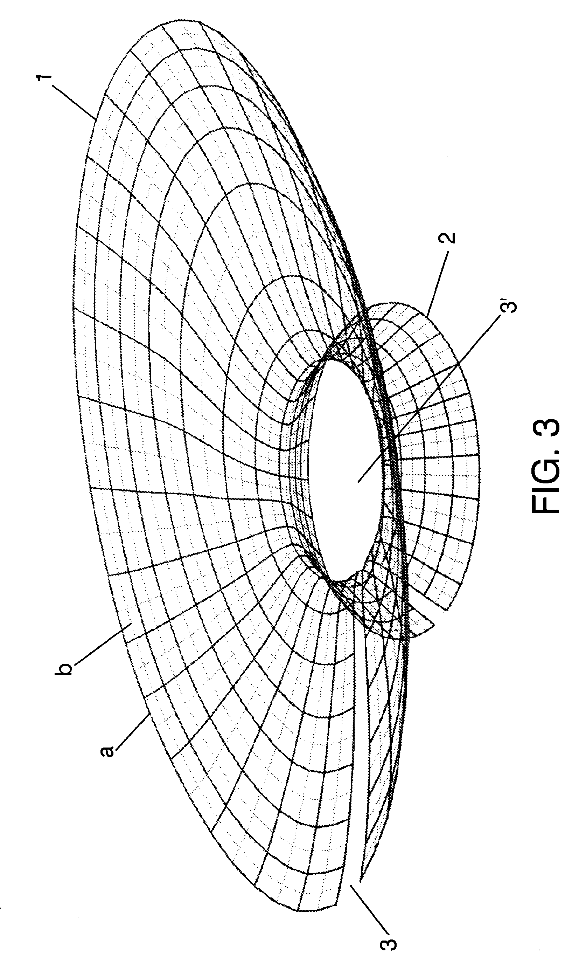

According to an additional embodiment, the mesh of the invention is made of a non-reabsorbing fibers framework (figure 3, a) and a reabsorbing tissue (figure 3, b).

In a second aspect, the invention refers to the surgical use of the mesh of the invention for the hernia treatment, particularly inguinal hernia.

### BRIEF DESCRIPTION OF THE DRAWINGS

In order to complement the description and with the object to help to a better understanding of the features of the invention, according to a preferred embodiment of it, a set of drawings is enclosed as an integral part of the said description, wherein the following is represented in an illustrative but not limitative way:
Figure 1 shows a lateral view of the mesh of the invention.
Figure 2 shows a view of the invention's mesh upper side (1), in which middle size standard measurements are detailed.
Figure 3 shows a view of the upper side of the mesh of the invention, in which the non-reabsorbing fibers (a) and the reabsorbing tissue (b), with an opening (3) and a central gap (3'), are highlighted.

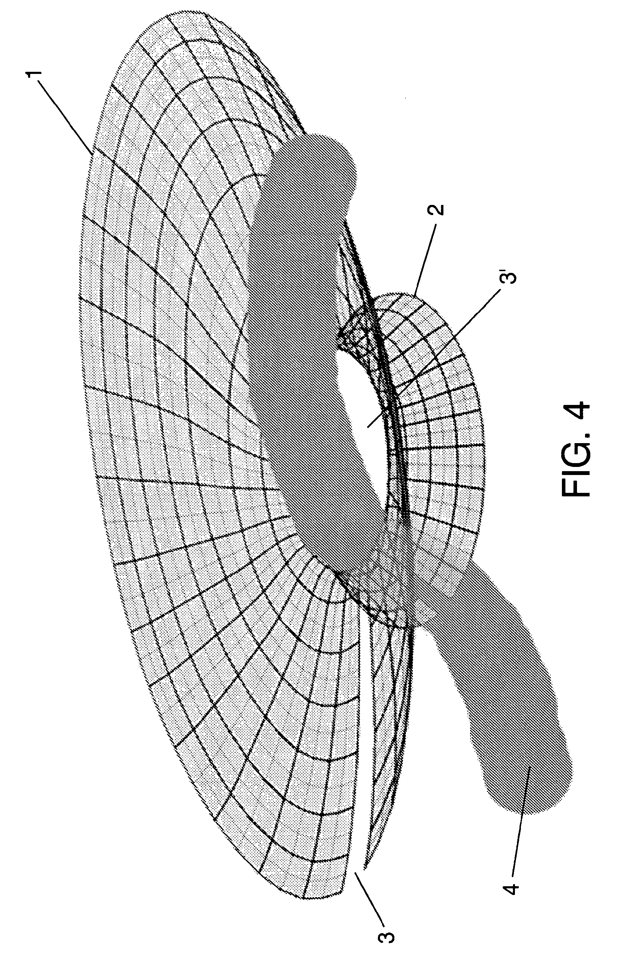

Figure 4 shows a view of the upper side of the mesh of the invention with the passing spermatic cord (4).

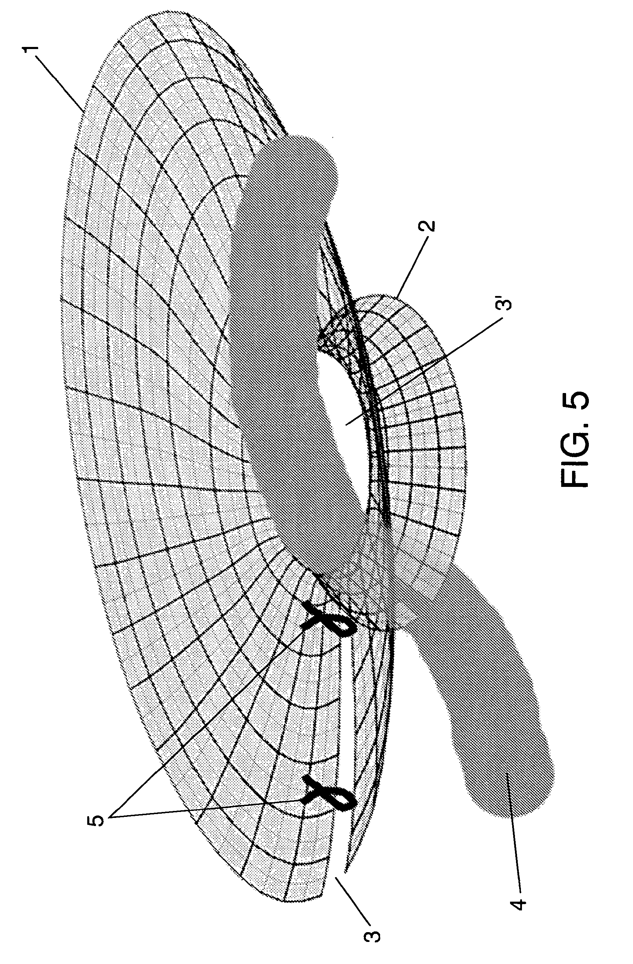

Figure 5 shows the closing of the mesh lateral opening with 2 sutures (5) that do not penetrate the tissues.

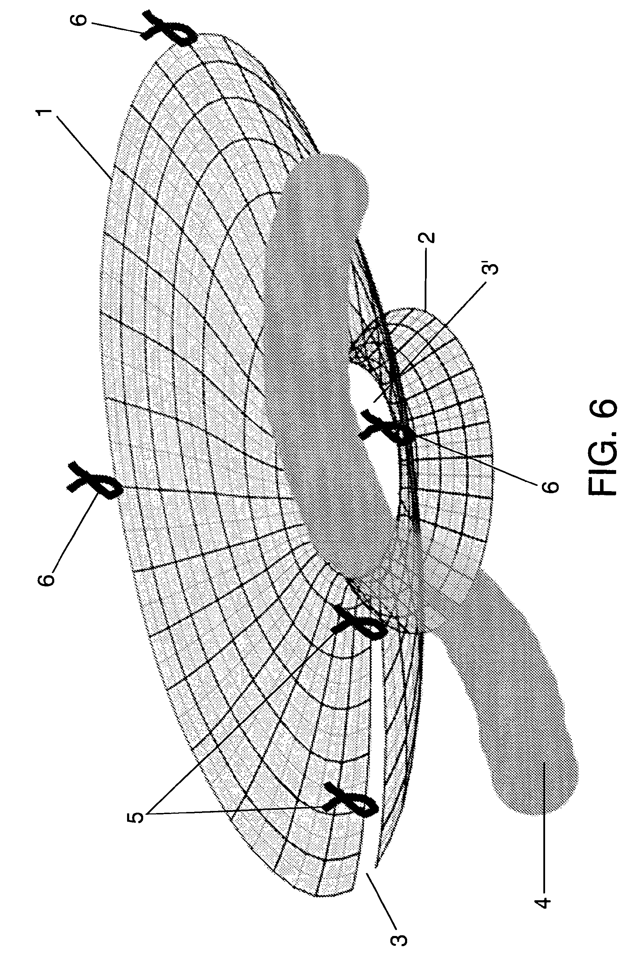

Figure 6 shows the mesh with the passing spermatic cord (4), the mesh lateral opening stitches (5) and other optional stitches (6) which join the mesh to the inguinal canal floor tissues in order to fix it.

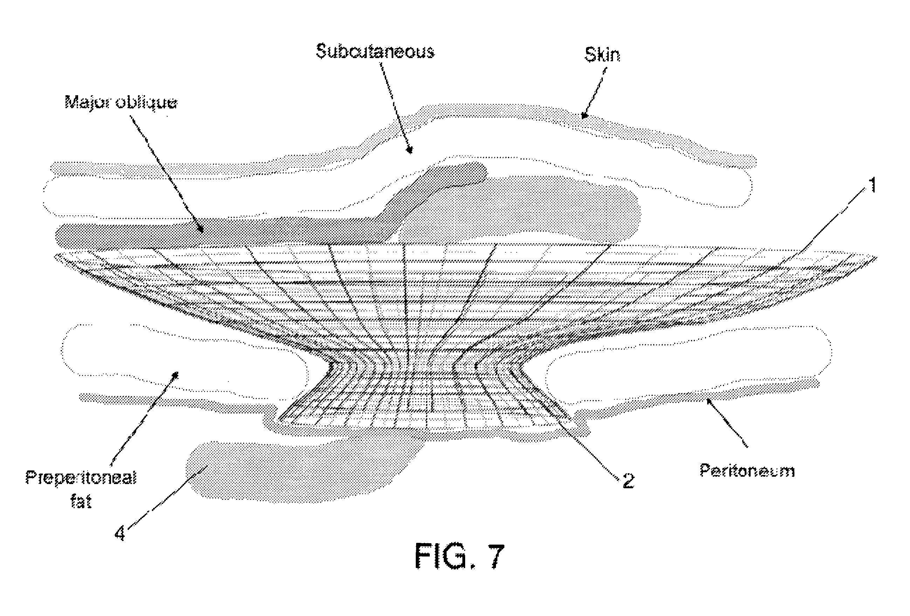

Figure 7 shows a lateral view of the mesh of the invention with the passing spermatic cord (4) and the layers that remain over and under the mesh.

### DETAILED DESCRIPTION OF THE INVENTION

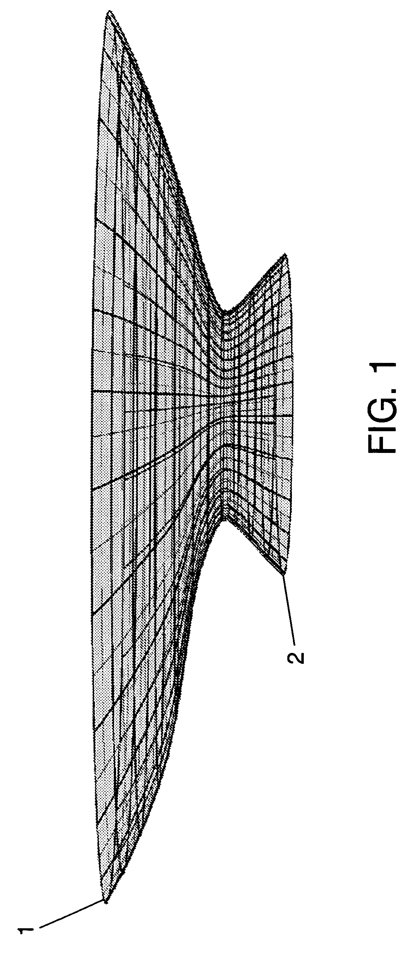

The mesh has two surfaces (1, 2) which form a catenoid structure (see figure 1). This particular three-dimensional structure makes possible to simultaneously repair the hernial defect in the preperitoneal space and the inguinal canal floor, as well as the inguinal orifice occlusion.

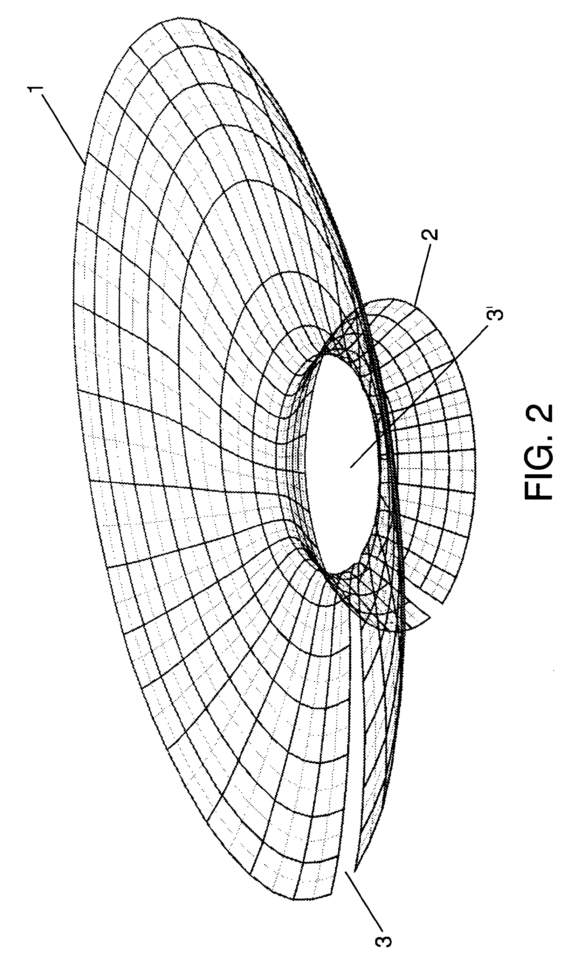

As seen in figure 2, the upper surface (1) is elliptical-shaped in order to fit to the inguinal canal floor shape and with dimensions suitable for its size. Given the structure of the mesh of the invention, it is important to highlight that it does not need any shape change to adapt it to the patient's anatomy. The inguinal wall dimensions vary among patients depending on their body structure but generally there are not big differences in surface and, consequently, it is not necessary to manufacture the mesh individually.

However, a certain number of standard sizes could be designed if necessary, so that the surgeon could choose the most appropriate one for each situation. In a particular embodiment, the ellipse's size has a major axis from 13 cm to 17 cm long and a minor axis from 8 cm to 12 cm long. According to a more particular embodiment, the ellipse's major axis is 15 cm long. In another more particular embodiment, the ellipse's minor axis is 10 cm long. According to an even more particular embodiment, the ellipse's major axis is 15 cm long and the ellipse's minor axis is 10 cm.

The lower surface (2) is circular-shaped and is located in the preperitoneal space. Just as it happens with the upper surface, the anatomical similarity amongst patients enables the design of the mesh with a particular standard lower surface size. In a particular embodiment, the lower surface diameter is from 4 cm to 6 cm long, more particularly is 5 cm long.

Likewise, according to a particular embodiment, the central gap (3') diameter is from 1cm to 2 cm long, more particularly is 1.5 cm long.

More specifically, the inventors of the present surgical mesh propose that just two or three predefined sizes could cater for practically any adult patient morphological characteristics.

Therefore, in a preferred embodiment, the invention is meant for a "large"-sized mesh with the following measurements:
- Upper side longitudinal axis 17 cm
- Upper side transverse axis 12 cm
- Lower side axis 6 cm
- Communicating orifice diameter 2 cm

According to another preferred embodiment, the invention is meant for a "medium"-sized mesh with the following measurements:
- Upper side longitudinal axis 15 cm
- Upper side transverse axis 10 cm
- Lower side axis 5 cm
- Communicating orifice diameter 1.5 cm

In another preferred embodiment, the invention is meant for a "small"-sized mesh with the following measurements:
- Upper side longitudinal axis 13 cm
- Upper side transverse axis 8 cm
- Lower side axis 4 cm
- Communicating orifice diameter 1 cm

The catenoid structure is interrupted by a non-connected lateral space (3) which will enable the spermatic cord (4) (men) or the round ligament (women) to pass without modifying or manipulating the mesh.

The mesh of the invention is compounded of a non-reabsorbing fibers framework (figure 3, a) and a reabsorbing tissue (figure 3, b) that shape a thin mesh with a very steady shape which perfectly adapts itself to the patient's stretching and dilatation movements caused by the body movement and the muscular contractions.

The mesh of the invention non-reabsorbing fibers (a) produce a solid retaining structure that guarantees the mesh' shape maintenance and stimulates the colonization by fibroblasts. These non-reabsorbing fibers (a) must be biocompatible and are appropriately selected from among the group of materials formed by polypropylene, Gore Tex^{®}, mixtures of them and mixtures with other biocompatible non-reabsorbing materials. The polypropylene and Gore Tex^{®} fibers have a monofilar structure and can be arranged in all the mesh' surface as well as in its external rim in order to give solidity with the least possible non-reabsorbing material. Besides, the high stress resistance of these fibers allows that the mesh is not deformed.

In the present invention, by biocompatible non-reabsorbing (or non-absorbing) fiber is meant the fiber which once implanted in the organism stays unaltered with the passage of time, it does not produce immune system response, it is not toxic by itself or by its degradation products, it is neither carcinogenic in the short term nor in the long term, and neither are its degradation products. Ths kind of fibers is widely well-known in the state of the art.

The mesh of the invention reabsorbing tissue (b) is made up of a tissue of absorbing fibers separated enough to allow free circulation of liquids, preventing the accumulation of sustances. Those fibers form a light tissue which is gradually reabsorbed by hydrolysis, with a progressive mass and stress resistance reduction. Adequate absorbing materials include homopolymers and copolymers of glycolate, of lactate, mixtures of them and mixtures with other biocompatible reabsorbing materials.

The mesh of the invention absorbing tissue is a weave of very low-weight fibers appropriately selected from among the group of materials formed by polyglactin 910, polyglycolic acid, polyglycolate, mixtures of them and mixtures with other biocompatible reabsorbing materials. Polyglactin 910 polyglycolate and polyglicolic acid are biodegradable polymers, widely used as material for the absorbing sutures synthesis. The absorbing tissue has pores, preferably of a diameter equal or higher than 1 millimeter to allow the easy passage of fluids and cellular elements, avoiding their retention and the formation of collections.

In the present invention, by biocompatible reabsorbing (or absorbing) fiber is meant the fiber which once implanted in the organism gradually reabsorbs without producing immune system response, it is not toxic by itself or by its degradation products, it is neither carcinogenic in the short term nor in the long term, and neither are its degradation products. This kind of fibers is widely well-known in the state of the art.

All the mesh of the invention' characteristics contribute to decrease the postoperative pain risks, the neuralgia caused by nervous entrapment and the quantity of foreign tissue that cause foreign body effects. Likewise, the mesh of the invention guarantees a good tolerance and that there is not any noticeable hardened area.

### Surgical example

Next, a surgical example is shown which complements the description in an illustrative but not limitative way, and highlights the easy implantation of the mesh of the invention.

With the patient in dorsal decubitus, the operation is started with a little incision (4-5 cm) on the skin to the level of the external inguinal ring. An incision is made in the subcutaneous adipose tissue up to the external oblique aponeurosis. The latter is cut about 3 centimeters in parallel to its fibers, opening the external inguinal ring and respecting the ilioinguinal and genitofemoral nerves. the anatomization of the spermatic cord or the round ligament is performed and the inguinal canal floor is released. The hernial sac is located and anatomized and it is reduced into the preperitoneal space.

The blunt dissection of the preperitoneal space through the internal inguinal orifice is performed by making a "pocket" able to place the mesh' lower surface (circle of about 4-5 centimeters of diameter). Next, the mesh' lower surface is placed in this preperitoneal space so that the hernial sac remains below it; the spermatic cord or the round ligament is passed through the mesh' lateral opening so that it stays leaning along the upper surface of the mesh. The upper side of the mesh is spread out on the inguinal canal floor. Close lateral opening of the mesh is closed with a single suture to keep it well fit around the spermatic cord or the round ligament.

To prevent later displacements, a suture is put in each of the mesh' upper surface longitudinal ends if it is desired. The major oblique muscle aponeurosis is sutured with anatomical-shaped reabsorbing suture. The subcutaneous cellular adipose tissue and the skin are sutured.

## Claims

1. Catenoid-shaped mesh comprising:
a) an elliptical-shaped upper side (1)
b) a circular-shaped lower side (2)
leaving a non-connected lateral space between both sides (3) and a central gap (3').

2. Mesh according to claim 1 comprising an elliptical-shaped upper side (1) with a major axis comprised from 13 cm to 17 cm.

3. Mesh according to claim 2 comprising an elliptical-shaped upper side (1) with a major axis is 13 cm, 15 cm or 17 cm long.

4. Mesh according to any of the claims 1-3 comprising an elliptical-shaped upper side (1) with a minor axis comprised from 8 cm to 12 cm.

5. Mesh according to claim 4 comprising an elliptical-shaped upper side (1) with a minor axis is 8 cm, 10 cm or 12 cm long.

6. Mesh according to any of the claims 1-5 comprising a circular-shaped lower side (2) with a diameter comprised from 4 cm to 6 cm.

7. Mesh according to claim 6 comprising a circular-shaped lower side (2) with a diameter is 4 cm, 5 cm or 6 cm long.

8. Mesh according to any of the claims 1-7 comprising a central gap (3') with a diameter comprised from 1cm to 2 cm.

9. Mesh according to claim 8 comprising a central gap (3') which diameter is 1 cm, 1.5 cm or 2 cm long.

10. Mesh according to any of the claims 1-9 comprising non-reabsorbing fibers (a) and a reabsorbing tissue (b).

11. Mesh according to claim 10 where the non-reabsorbing fibers (a) are selected from among the group of materials formed by polypropylene, Gore Tex^{®}, mixtures of them and mixtures with other biocompatible non-reabsorbing materials.

12. Mesh according to claim 11 where the non-reabsorbing fibers (a) are selected from among the group of materials formed by polypropylene, Gore Tex^{®} and mixtures of them.

13. Mesh according to any of the claims 10-12 where the reabsorbing tissue (b) is selected from among the group of materials formed by homopolymers and copolymers of glycolate, of lactate, mixtures of them and mixtures with other biocompatible reabsorbing materials.

14. Mesh according to claim 13 where the reabsorbing tissue (b) is selected from among the group of materials formed by polyglactin 910, polyglycolic acid, polyglycolate and mixtures of them.

## Patentansprüche

1. Kettenlinienförmiges Maschennetz mit:
a) einer oberen Seite in Ellypsenform (1),
b) einer unteren Seite in Kreisform (2),
das einen seitlichen unverbundenen Raum (3) auf beiden Seiten und einen Zwischenraum in der Mitte (3') frei lässt.

2. Maschennetz nach Anspruch 1 mit einer oberen Seite in Ellypsenform (1), deren große Achse 13 cm - 17 cm lang ist.

3. Maschennetz nach Anspruch 2 mit einer oberen Seite in Ellypsenform (1), deren große Achse 13 cm, 15 cm oder 17 cm lang ist.

4. Maschennetz nach einem der Ansprüche 1 - 3 mit einer oberen Seite in Ellypsenform (1), deren kleine Achse 8 cm - 12 cm lang ist.

5. Maschennetz nach Anspruch 4 mit einer oberen Seite in Ellypsenform (1), deren kleine Achse 8 cm, 10 cm oder 12 cm lang ist.

6. Maschennetz nach einem der Ansprüche 1 - 5 mit einer unteren Seite in Kreisform (2), deren Durchmesser 4 cm - 6 cm lang ist.

7. Maschennetz nach Anspruch 6 mit einer unteren Seite in Kreisform (2), deren Durchmesser 4 cm, 5 cm oder 6 cm lang ist.

8. Maschennetz nach einem der Ansprüche 1 - 7 mit einem Zwischenraum in der Mitte (3'), dessen Durchmesser 1 cm - 2 cm lang ist.

9. Maschennetz nach Anspruch 8 mit einem Zwischenraum in der Mitte (3'), dessen Durchmesser 1 cm, 1,5 cm oder 2 cm lang ist.

10. Maschennetz nach einem der Ansprüche 1 - 9 mit nicht absorbierenden Fasern (a) und einem absorbierenden Gewebe (b).

11. Maschennetz nach Anspruch 10, wobei die nicht absorbierenden Fasern (a) aus der Materialgruppe ausgewählt werden, die aus Polypropylen, Gore Tex®, deren Mischungen und aus Mischungen mit anderen biokompatiblen nicht absorbierenden Materialien besteht.

12. Maschennetz nach Anspruch 11, wobei die nicht absorbierenden Fasern (a) aus der Materialgruppe ausgewählt werden, die aus Polypropylen, Gore Tex® und deren Mischungen besteht.

13. Maschennetz nach einem der Ansprüche 10 - 12, wobei das absorbierende Gewebe (b) aus der Materialgruppe ausgewählt wird, die aus Glykolat-Homopolymeren und -Kopolymeren, Laktat, deren Mischungen und Mischungen mit anderen biokompatiblen absorbierenden Materialien besteht.

14. Maschennetz nach Anspruch 13, wobei das absorbierende Gewebe (b) aus der Materialgruppe ausgewählt wird, die aus Polyglaktin 910, Polyglykolsäure, Polyglykolat und deren Mischungen besteht.

## Revendications

1. Filet à mailles en forme de caténoïde, comprenant :
a) un côté supérieur de forme elliptique (1),
b) un côté inférieur de forme circulaire (2),
laissant un espace latéral non relié (3) s'étendant sur les deux côtés et un intervalle central (3').

2. Filet à mailles selon la revendication 1, comprenant un côté supérieur de forme elliptique (1) dont le grand axe a une longueur de 13 cm à 17 cm.

3. Filet à mailles selon la revendication 2, comprenant un côté supérieur de forme elliptique (1) dont le grand axe a une longueur de 13 cm, 15 cm ou 17 cm.

4. Filet à mailles selon l'une quelconque des revendications 1 à 3, comprenant un côté supérieur de forme elliptique (1) dont le petit axe a une longueur dans la plage de 8 cm à 12 cm.

5. Filet à mailles selon la revendication 4, comprenant un côté supérieur de forme elliptique (1) dont le petit axe a une longueur de 8 cm, 10 cm ou 12 cm.

6. Filet à mailles selon l'une quelconque des revendications 1 à 5, comprenant un côté inférieur de forme circulaire (2) dont le diamètre a une longueur dans la plage de 4 cm à 6 cm.

7. Filet à mailles selon la revendication 6, comprenant un côté inférieur de forme circulaire (2) dont le diamètre a une longueur de 4 cm, 5 cm ou 6 cm.

8. Filet à mailles selon l'une quelconque des revendications 1 à 7, comprenant un intervalle central (3') dont le diamètre a une longueur de 1 cm à 2 cm.

9. Filet à mailles selon la revendication 8, comprenant un intervalle central (3') dont le diamètre a une longueur de 1 cm, 1,5 cm ou 2 cm.

10. Filet à mailles selon l'une quelconque des revendications 1 à 9, comprenant des fibres non absorbantes (a) et un tissu absorbant (b).

11. Filet à mailles selon la revendication 10, où les fibres non absorbantes (a) sont choisies dans le groupe de matériaux formé par le polypropylène, le Gore Tex®, des mélanges de ceux-ci et des mélanges avec d'autres matériaux non absorbants biocompatibles.

12. Filet à mailles selon la revendication 11, où les fibres non absorbantes (a) sont choisies dans le groupe de matériaux formé par le polypropylène, le Gore Tex® et des mélanges de ceux-ci.

13. Filet à mailles selon l'une quelconque des revendications 10 à 12, où le tissu absorbant (b) est choisi dans le groupe de matériaux formé par les homopolymères et copolymères de glycolate, le lactate, des mélanges de ceux-ci et des mélanges avec d'autres matériaux absorbants biocompatibles.

14. Filet à mailles selon la revendication 13, où le tissu absorbant (b) est choisi dans le groupe de matériaux formé par la polyglactine 910, l'acide polyglycolique, le polyglycolate et des mélanges de ceux-ci.
